# EUROPÄISCHE PATENTANMELDUNG

(11) **EP 1 922 926 A1**
(43) Veröffentlichungstag der Anmeldung: **21.05.2008**
(21) Anmeldenummer: 07118755.3
(22) Anmeldetag: 18.10.2007
(51) Int. Cl.: A01N 37/18, A61Q 11/00, C07C 235/06

(54) **Antimikrobielle Zusammensetzungen**

(30) Priorität: 14.11.2006 DE 102006053500
(71) Anmelder: Evonik Goldschmidt GmbH, 45127 Essen (DE)
(72) Erfinder: Wenk, Hans Henning, 45138, Essen (DE); Allef, Petra, 47807, Krefeld (DE); Bergfried, Stefan, 45134, Essen (DE); Farwick, Mike, 45138, Essen (DE); Grüning, Burghard, 45134, Essen (DE)

(57) **Zusammenfassung**

Gegenstand der Erfindung sind Verbindungen der allgemeinen Formel 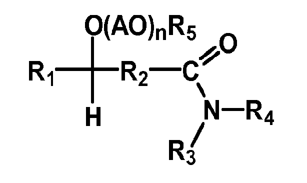 und deren Verwendung zur Kontrolle von Mikroorganismen.

## Beschreibung

Die vorliegende Erfindung betrifft antimikrobielle Zusammensetzungen sowie deren Verwendung in kosmetischen Zubereitungen. Antimikrobielle Wirkstoffe finden breite Anwendung in kosmetischen Desodorantien, Antischuppen- und Antiakne-Formulierungen, Fußpflege- und Intimhygienemitteln sowie Mundhygiene- und Zahnpflegeprodukten.

Körpergeruch entsteht vor allem, wenn der zunächst geruchlose Schweiß durch Mikroorganismen auf der Haut zersetzt wird. Erst die mikrobiellen Abbauprodukte verursachen den unangenehmen Schweißgeruch. Dieser entsteht insbesondere dort, wo eine hohe Dichte an Schweißdrüsen besteht und zudem eine hohe Dichte an geruchserzeugenden Keimen vorliegt, wie z.B. unter den Achseln.

Auch bestimmte Hauterkrankungen stehen in Zusammenhang mit übermäßigem Wachstum unerwünschter Mikroorganismen auf der Haut. So wird Akne unter anderem durch unkontrollierte Vermehrung des anaeroben Hautbakteriums Propionibacterium acnes verursacht. Schuppen werden u.a. in Zusammenhang mit dem Pilz Malassezia Furfur gebracht.

Im Bereich der Mundhygiene spielen Mikroorganismen, z.B. bei der Entstehung von Karies und Zahnbelag, eine wesentliche Rolle.

### Stand der Technik

Die Wirkung von Desodorantien kann auf unterschiedlichen Mechanismen beruhen, die gegebenenfalls auch kombiniert werden können:
- Vermeidung von Schweißbildung durch Antiperspirantien, wie z.B. Aluminiumchlorhydrat (ACH) oder Aluminium Zirkonium Tetrachlorohydrex GLY,
- Verhinderung der Geruchsbildung durch Kontrolle von geruchsbildenden Mikroorganismen (insbesondere coryneforme),
- Verhinderung der Geruchsbildung durch Inhibition mikrobieller Enzyme, die für die Geruchsbildung aus Schweiß verantwortlich sind,
- Bindung von Gerüchen, die den Schweißgeruch verursachen, durch Stoffe z.B. durch Zinkverbindungen wie Zinkricinoleat oder dessen Salze, oder durch Cyclodextrine,
- Überdeckung des Schweißgeruches durch Duftstoffe.

### Antimikrobielle Wirkstoffe

Die Verwendung antimikrobieller Wirkstoffe in kosmetischen Formulierungen, insbesondere in kosmetischen Desodorantien, ist aus dem Stand der Technik hinlänglich bekannt.

Anwendung findet z.B. Triclosan (5-Chlor-2-(2,4-dichlorphenoxy)-phenol), das antimikrobielle Wirkung gegen ein breites Spektrum von Mikroorganismen zeigt. Aufgrund der Breitbandwirkung wirkt sich Triclosan nachteilig auf die Mikroflora der Haut aus. Außerdem besteht aufgrund der spezifischen Wirkungsweise die Gefahr der Bildung von Resistenzen.

DE 42 40 674 beansprucht eine Kombination von Glycerinmonoalkylethern der allgemeinen Formel

R-O-CH₂-CHOH-CH₂OH

wobei R eine verzweigte oder unverzweigte C₆₋₁₈ Alkylgruppe ist, mit einem oder mehreren weiteren desodorierenden Wirkstoffen als Deo-Zusammensetzung.

Nachteilig bei diesen Verbindungen ist, dass sie in wässrigen Systemen nur eine geringe Löslichkeit aufweisen und in solchen Systemen nur in Kombination mit Solubilisatoren formuliert werden können. Außerdem wirken diese Substanzen hemmend oder keimreduzierend auch auf typische Vertreter der normalen Hautflora, z.B. Staphylococcus epidermidis, wodurch das mikrobiologische Gleichgewicht auf der Haut nachhaltig gestört werden kann.

WO 02/30383 beansprucht antimikrobielle Desodorantien, die als Wirkstoff einen Übergangsmetallchelator enthalten.

DE OS 42 37 081 beschreibt kosmetische Desodorantien, gekennzeichnet durch einen wirksamen Gehalt an Monocarbonsäureestern des Di- oder Triglycerins. Diese Verbindungen weisen eine spezifische Hemmung des Wachstums geruchsbildender Keime auf, sind jedoch aufgrund der Esterbindung nur in Formulierungen mit einem eingeschränkten pH-Bereich langzeitstabil.

EP 0 262 587 beansprucht N-Octyl- und N-Decyl-salicylsäureamid unter anderem als antimikrobiellen Wirkstoff in Körperdesodorantien. Andere Derivate, insbesondere solche, die auf aliphatischen Carbonsäuren basieren, oder alkoxylierte Derivate werden nicht beschrieben.

### Amide in kosmetischen Formulierungen

Einfache N-substituierte Milchsäureamide sind seit langem bekannt. Beispielsweise beschreibt Ratchford die Synthese von N-Alkyl-, N-Aryl- und N-Alkenylamiden der Milchsäure ("Preparation of N-substituted lactamides by aminolysis of methyl lactate, J. Org. Chem. 1950, 15, 317 bis 325; "Preparation and properties of N-alkyllactamides", J. Org. Chem. 1950, 15, 326 bis 332) durch Umsetzung von Milchsäure oder Methyllactat mit Alkylaminen.

JP2005-060457 beschreibt Verbindungen der allgemeinen Formel

R₁-NH-CO-R₂

wobei R₁ ein linearer oder verzweigter C₆₋₂₁ Alkylrest, R₂ eine Methyl-, 1-Hydroxyethyl- oder 1-Hydroxy-1-methylethylgruppe ist, als Verdicker in kosmetischen Formulierungen. Eine antimikrobielle Wirkung der Produkte ist nicht beschrieben.

DE OS 26 31 284 beansprucht kosmetische Mittel mit einem Gehalt an Amiden von Hydroxyalkancarbonsäuren der allgemeinen Formel

R₁-CHOH-R₂-CONR₃R₄

wobei R₁=C₁₋₄ Alkyl, R₂ eine direkte Bindung oder C₁₋₃ Alkylen (ggf. alkyl- oder hydroxysubstituiert), R₃ und/oder R₄ unabhängig voneinander C₁₋₄ Alkyl oder C₂₋₆ Hydroxyalkyl mit 1 bis 5 OH-Gruppen bedeuten.

In den beanspruchten kosmetischen Mitteln dienen die Amide als Feuchthaltemittel. Eine antimikrobielle Wirkung ist nicht erwähnt. Außerdem sind weder längerkettige noch alkoxylierte Derivate beschrieben.

US 3,916,003 beschreibt nichtionische Tenside der Formel 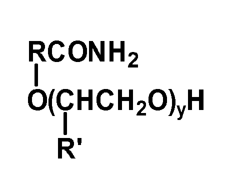 wobei R ein aliphatischer Kohlenwasserstoffrest mit 17 C-Atomen, die Polyoxyalkylenkette an Position 12 der C-Kette gebunden, R' ein Wasserstoffatom oder ein kurzkettiger Alkylrest sowie y eine Zahl von 1 bis 50 ist. Weder N-alkylierte noch kürzerkettige Derivate sind beschrieben.

US 4,851,434 beansprucht Verbindung der allgemeinen Struktur 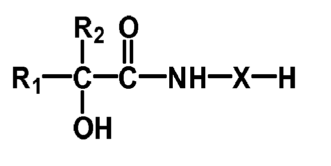 wobei R₁ und R₂ H oder kurze Alkylreste sind und X einer der Gruppen (CH₂CH₂O)ₙ, (CH2CH(CH₃)O)ₙ, (CH₂CH₂CH(CH₃)O)ₙ oder CH₂(CHOH)₄CH₂O entspricht. Die Verbindungen werden als Feuchthaltemittel in kosmetischen Formulierungen beschrieben. Die Substanzen unterscheiden sich grundsätzlich von den in dieser Anmeldung beschriebenen Stoffen, da diese Polyalkylenoxidgruppen am Amidstickstoff enthalten (nicht am OH-Sauerstoff) und außerdem keine einfachen N-Alkylreste enthalten. Des Weiteren werden auch hier keine antimikrobiellen oder bakteriostatischen Eigenschaften beschrieben.

### Aufgabenstellung

Aus dem Stand der Technik ergab sich demzufolge die Aufgabe, Verbindungen zur Verfügung zu stellen, die eine selektive Kontrolle unerwünschter Mikroorganismen ermöglichen, insbesondere solcher, die auf der Haut angesiedelt sind. Zusätzlich sollten die Verbindungen stabil in unpolaren und polaren (insbesondere wässrigen) Systemen sein, eine gute Hautverträglichkeit aufweisen sowie gute Formulierbarkeit in gängigen Anwendungsformen, insbesondere kosmetischen Formulierungen, zeigen.

Unter "Kontrolle" ist dabei insbesondere eine Konstanthaltung oder Reduktion der Keimzahl durch Hemmung des Wachstums oder Abtötung der Organismen zu verstehen. "Selektiv" bedeutet in diesem Sinne, dass die kontrollierende Wirkung auf unerwünschte (z.B. geruchsbildende) Keime stärker ausgeprägt ist, als die auf Mikroorganismen der normalen Hautflora. Zu den unerwünschten Keimen zählen beispielsweise Corynebacterium xerosis, Propionibacterium acnes, Malassezia furfur, ohne jedoch auf diese beschränkt zu sein. Unter "Mikroorganismen" sind insbesondere Bakterien und Pilze (inkl. Hefen) zu verstehen. Der Ausdruck "Formulierungen" umfasst sogenannte "leave-on" Produkte wie Cremes, Lotionen, Pump- oder Aerosolsprays, Wischtücher, Deosticks und Roll-on Formulierungen; "rinse-off" Produkte wie Shampoos, Duschgele, Flüssigseifen, Haarspülungen und -konditionierer; Mundhygieneprodukte wie Mundspüllösungen oder Zahnpasten; Reinigungsprodukte wie Spülmittel, Haushaltsreiniger (Boden-, Küchen-, Badreiniger), ohne jedoch auf diese beschränkt zu sein.

Übliche Desodorantien werden vor allem in Form von Roll-On Spendern, Aerosolen, Pumpsprays, Deosticks, Trockendeosprays oder Wischtüchern, sog. "wipes", angeboten. Antimikrobielle Inhaltsstoffe werden darin entweder als alleiniger Wirkstoff oder in Kombination mit anderen als Antiperspirantien oder Desodorantien wirksamen Stoffen wie z.B. Triclosan, Ethylhexylglycerin, Aluminiumchlorohydrat, Aluminium Zirconium Tetrachlorohydrex GLY, Aluminium Zirconium Pentachlorohydrat, Farnesol, Polyglycerincaprinat oder -caprylat, Triethylcitrat, Penta(carboxymethyl) diethylenetriamine (pentetic acid), Pentylenglykol, Propylenglykol, Ethanol, Zinkricinoleat, Cyclodextrine oder Zinkoxid angewendet.

Das Spektrum der Formulierungsbasen reicht dabei von festen Formulierungen über flüssige oder cremeartige O/W oder W/O Emulsionen bis zu wässrigen, alkoholischen oder ölhaltigen flüssigen Systemen. Außerdem decken die Formulierungen einen großen pH-Bereich ab, der von etwa 3 bis etwa 9 reichen kann.

Hieraus ergeben sich nicht nur hohe Anforderungen an die Stabilität des Wirkstoffes (insbesondere soll unter den genannten Bedingungen keine Hydrolyse oder Alkoholyse stattfinden), sondern auch an die Wirksamkeit, die im gesamten pH-Bereich der möglichen kosmetischen Formulierungen möglichst konstant sein soll.

### Erfindung

Es hat sich nun überraschenderweise gezeigt, dass Verbindungen der allgemeinen Formel (I) die gestellten Aufgaben in ausgezeichneter Weise erfüllen.

Ein Gegenstand der Erfindung sind Verbindungen der allgemeinen Formel (I) 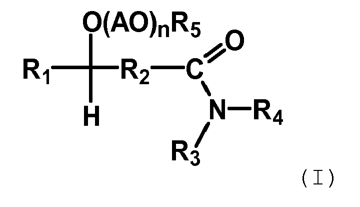 worin die Substituenten und Indices die folgende Bedeutung haben:
- R₁ =: H oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen, vorzugsweise 1 bis 10 C-Atomen, insbesondere 1 bis 5 C-Atomen,
- R₂ =: direkte Bindung oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen, vorzugsweise 1 bis 6 C-Atomen, insbesondere 1 bis 4 C-Atomen,
- R₃, R₄=: unabhängig voneinander H oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen, vorzugsweise 4 bis 12 C-Atomen, insbesondere 6 bis 10 C-Atomen,
- R₅ =: H oder C₁-C₄-Acylrest,
- AO =: mindestens ein Rest, ausgesucht aus der Gruppe -CH₂CH₂O-, -CH₂CHR₆O-, -CHR₆CH₂O-, -CH₂CH₂OHCH₂O- mit R₆=C₁-C₄-Alkyl,
- n =: 1 bis 20, vorzugsweise 1 bis 10, insbesondere 2 bis 5, bezogen auf den Mittelwert aller Oligomere,
mit der Maßgabe, dass R₃ und R₄ nicht gleichzeitig H sind und die Summe der C-Atome in R₃ und R₄ nicht größer als 18 ist und dass die Summe der C-Atome in R₁, R₂, R₃ und R₄ 36 nicht überschreitet.

Ein weiterer Gegenstand der Erfindung ist die Verwendung von Verbindungen der allgemeinen Formel (Ia) 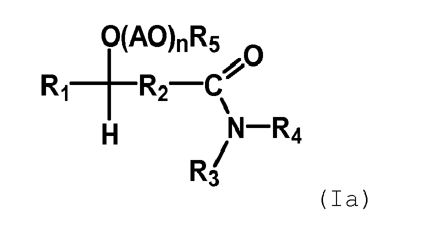 worin die Substituenten und Indices die folgende Bedeutung haben:
- R₁ =: H oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen, vorzugsweise 1 bis 10 C-Atomen, insbesondere 1 bis 5 C-Atomen,
- R₂ =: direkte Bindung oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen, vorzugsweise 1 bis 6 C-Atomen, insbesondere 1 bis 4 C-Atomen,
- R₃,R₄=: unabhängig voneinander H oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen, vorzugsweise 4 bis 12 C-Atomen, insbesondere 6 bis 10 C-Atomen,
- R₅ =: H oder C₁-C₄-Acylrest,

- AO =: mindestens ein Rest, ausgesucht aus der Gruppe -CH₂CH₂O-, -CH₂CHR₆O-, -CHR₆CH₂O-, -CH₂CH₂OHCH₂O- mit R₆=C₁-C₄-Alkyl,
- n =: 0 bis 20, vorzugsweise 0 bis 10, insbesondere 0 bis 5 bezogen, auf den Mittelwert aller Oligomere,
mit der Maßgabe, dass R₃ und R₄ nicht gleichzeitig H sind und die Summe der C-Atome in R₃ und R₄ nicht größer als 18 ist und dass die Summe der C-Atome in R₁, R₂, R₃ und R₄ 36 nicht überschreitet,
zur Kontrolle von Mikroorganismen.

Erfindungsgemäß verwendbare Verbindungen sind beispielsweise Verbindungen der allgemeinen Formel (I) auf der Basis von Glycolsäure, Hydroxybuttersäuren, Hydroxyvaleriansäuren, Hydroxycapronsäuren und insbesondere Milchsäure.

Erfindungsgemäß sind weiterhin Verbindungen bevorzugt, in denen, wenn n=0 ist, R₅=H, R₁=CH₃, R₂ eine direkte Bindung, R₃=H und R₄=C₄-C₁₂-, vorzugsweise C₈-Alkylrest, ist.

Erfindungsgemäß bevorzugt sind weiterhin Verbindungen, in denen n≥1, R₅=H oder Acyl, R₁=CH₃, R₂ eine direkte Bindung, R₃=H und R₄=C₄-C₁₂-, vorzugsweise C₈-Alkylrest, ist.

Insbesondere ermöglichen diese Verbindungen eine selektive Kontrolle unerwünschter Mikroorganismen auf der Haut und Hautanhangsgebilden, ohne die normale vorhandene Flora zu stark zu beeinflussen.

Die Verbindungen sind dabei stabil gegen Hydrolyse über einen großen pH-Bereich und lassen sich in verschiedene kosmetische Standardformulierungen problemlos einarbeiten. Darüber hinaus weist die Wirksamkeit keine nennenswerte Abhängigkeit vom pH-Wert auf, was bezüglich einer möglichst universellen Anwendbarkeit ein weiterer Vorteil ist.

Die Herstellung nicht alkoxylierter Milchsäurederivate durch Amidierung von Milchsäure oder Alkyllactaten mit Alkylaminen ist in der Literatur hinreichend beschrieben (vgl. z.B. "Preparation of N-substituted lactamides by aminolysis of methyl lactate, J. Org. Chem. 1950, 15, 317-325; "Preparation and properties of N-alkyllactamides", J. Org. Chem. 1950, 15, 326-332).

Entsprechend können auch Amide anderer Hydroxycarbonsäurederivate durch Reaktion der Carbonsäuren oder ihrer Alkylester oder Lactone mit primären oder sekundären Aminen hergestellt werden.

Als Hydroxycarbonsäureeinheiten können z.B. Milchsäure, Glycolsäure, 2-Hydroxybuttersäure, 4-Hydroxybuttersäure, 6-Hydroxyhexansäure, 2,2-Dimethylolpropionsäure, 3,3-Dimethyl-2,4-dihydroxybuttersäure oder deren C₁-C₄-Alkylester eingesetzt werden, wobei Milchsäure oder Milchsäureethylester besonders bevorzugt sind. Ebenso können z.B. die Lactone von 4-Hydroxybuttersäure oder 6-Hydroxyhexansäure verwendet werden.

Als Amine kommen primäre oder sekundäre aliphatische Amine wie z.B. n-Hexyl-, n-Octyl, n-Decylamin, n-Dodecylamin, 2-Ethylhexylamin, Ethanolamin oder Diethanolamin in Frage,
wobei acyclische, primäre aliphatische Amine (verzweigt oder unverzweigt) und insbesondere n-Hexylamin, n-Octylamin, n-Decylamin, n-Dodecylamin oder 2-Ethylhexylamin bevorzugt sind.

Die nicht alkoxylierten Hydroxycarbonsäureamide können durch Reaktion mit einem oder mehreren Alkylenoxiden oder Glycidol (insbesondere Ethylenoxid oder Propylenoxid oder Gemische aus beiden) bei erhöhter Temperatur und unter Druck, gegebenenfalls in Gegenwart geeigneter Katalysatoren, zu den entsprechenden alkoxylierten Derivaten umgesetzt werden. Hierbei entsteht immer eine Verteilung verschiedener Polyalkylenoxidketten, deren Mittelwert durch den eingesetzten molaren Überschuss an Alkylenoxid gesteuert werden kann. Es ist dem Fachmann geläufig, dass die Oxyalkyleneinheiten blockweise verteilt sein können oder in Form eines Gemisches mit einer im Wesentlichen durch statistische Gesetze geregelten Verteilung vorliegen. Außerdem entsteht als Nebenprodukt immer ein Anteil Polyalkylenoxid, der vorzugsweise im Produkt verbleibt. Der eingesetzte Katalysator kann nach der Reaktion durch geeignete Zusätze neutralisiert werden. Bei Verwendung von KOH oder NaOH als Katalysator sind beispielsweise organische oder anorganische Säuren geeignet, besonders vorteilhaft sind organische Säuren wie Zitronensäure oder Milchsäure und diejenigen Hydroxycarbonsäuren, die in Form der Amide in der Reaktion eingesetzt wurden.

Die erfindungsgemäßen Verbindungen können in einer Vielzahl von Formulierungen für Anwendungen in Haushalt, Industrie, Pharmazie und Kosmetik eingesetzt werden. Besonders geeignet sind sie als wirksame Komponenten in Desodorantien, die in Form von Aerosolsprays, Pumpsprays, Roll-On Formulierungen, Deo-Sticks, W/O- oder O/W-Emulsionen (z.B. Cremes oder Lotionen) oder Wischtüchern vorliegen können. Mit verwendet werden können in diesen Formulierungen die aus dem Stand der Technik bekannten Wirkstoffe/Wirkstoffkombinationen, wie z.B. Triclosan, Ethylhexylglycerin, Aluminiumchlorohydrat, Aluminium Zirconium Tetrachlorohydrex GLY, Aluminium Zirconium Pentachlorohydrat, Farnesol, Polyglycerincaprinat oder -caprylat, Triethylcitrat, Penta(carboxymethyl)diethylenetriamine (pentetic acid), Pentylenglykol, Propylenglykol, Ethanol, Zinkricinoleat, Cyclodextrine oder Zinkoxid.

Die Anwendung ist jedoch nicht auf den Einsatz in Desodorantien beschränkt, sondern kann überall dort vorteilhaft sein, wo eine Bekämpfung von Mikroorganismen oder von deren Wachstum erwünscht ist, wie z.B. in Intimhygieneartikeln, Antiakne- oder Antischuppenprodukten, die in Form der gängigen leave-on oder rinse-off Formulierungen vorliegen können, wie z.B. Cremes, Lotionen, Shampoos, Waschlösungen, Haarspülungen, Wischtüchern und ähnlichen Formulierungen.

Für Antiakneprodukte können die erfindungsgemäßen Stoffe gegebenenfalls auch in Kombination mit bekannten Antiaknewirkstoffen, wie z.B. Dibenzoylperoxid, Salicylsäure, Phytosphingosin, Tretinoin, Isotretinoin oder Pflanzenextrakten, eingesetzt werden. Ebenso können im Falle von Antischuppenprodukten Kombinationen mit bekannten Antischuppenwirkstoffen, wie z.B. Climbazol, Zinkpyrethion, Selenverbindungen (beispielsweise Selensulfid), Piroctone Olamine (Octopirox) oder Pflanzenextrakten, verwendet werden.

Auch im Bereich von Mundhygieneprodukten können die erfindungsgemäßen Stoffe verwendet werden, wobei sich hier insbesondere der Einsatz in Mundspüllösungen oder Zahnpasten empfiehlt.

Sie können auch in Kombination mit anderen Wirkstoffen, die in Bereichen Wirksamkeitslücken aufweisen, in denen die erfindungsgemäßen Stoffe wirksam sind, für diesen Zweck eingesetzt werden. So kann der Einsatz von Konservierungsmitteln verringert oder gegebenenfalls sogar vollständig auf klassische Konservierungsmittel verzichtet werden.

### Beispiele

| | | |
|---|---|---|
| Erklärung der eingesetzten Rohstoffe: | | |

| Handelsname | INCI-Bezeichnung | Hersteller |
|---|---|---|
| TEGO® SML 20 | Polysorbate 20 | Goldschmidt GmbH |
| ABIL® B 8832 | Bis-PEG/PPG-20/20 Dimethicone | Goldschmidt GmbH |
| Tegosoft® GC | PEG-7 Glyceryl Cocoate | Goldschmidt GmbH |
| Reach® 501 | Aluminium Chlorohydrate | Reheis, Inc. |
| Kathon^{™} CG | Methylchloroisothiazol inone and Methylisothiazolinone | Rohm and Haas |
| Tegosoft® M | Isopropyl Myristate | Goldschmidt GmbH |
| Allantoin | Allantoin | Fluka |
| Panthenol | Panthenol | Roche |
| Walocel® HM 4000 | Hydroxypropylmethylcel lulose | Wolff Cellulosics |
| Tylose® H4000 | Hydroxyethylcellulose | Clariant |
| Teginacid® H | Glyceryl Stearate and Ceteth-20 | Goldschmidt GmbH |
| TEGO® Alkanol 18 | Stearyl Alcohol | Goldschmidt GmbH |
| Tegosoft® Liquid | Cetearyl Ethylhexanoate | Goldschmidt GmbH |
| Tegosoft® CT | Caprylic/Capric Triglyceride | Goldschmidt GmbH |
| TEGO® Alkanol S2P | Steareth-2 | Goldschmidt GmbH |
| TEGO® Alkanol S20P | Steareth-20 | Goldschmidt GmbH |
| Varonic® APS | PPG-11 Stearyl Ether | Goldschmidt GmbH |
| ABIL® 350 | Dimethicone | Goldschmidt GmbH |
| Tegosoft® PBE | PPG-14 Butyl Ether | Goldschmidt GmbH |
| Tegosoft® P | Isopropyl Palmitate | Goldschmidt GmbH |
| TEGO® Alkanol L4 | Laureth-4 | Goldschmidt GmbH |
| Micro-Dry® Ultrafine | Aluminium Chlorohydrate | Reheis, Inc. |

### Beispiel 1

### Herstellung von Milchsäure-N-octylamid

In einem Rundkolben mit Destillationsapparatur werden 388 g (3 mol) n-Octylamin innerhalb von zwei Stunden unter Rühren und Stickstoffeinleitung zu 473 g (4 mol) Ethyllactat zugegeben. Nach vollendeter Zugabe wird die Temperatur auf 90°C erhöht und das entstehende Ethanol destilliert. Die Temperatur wird auf 140°C erhöht und für eine Stunde gehalten. Anschließend wird der Druck auf 50 mbar verringert und nicht umgesetztes Ethyllactat destilliert. Es werden 585 g Milchsäureoctylamid als klare, schwach rosafarbene Flüssigkeit erhalten.
¹H-NMR (400 MHz, CDCl₃) : 0,8 ppm (3H), 1,2 ppm (10H), 1,4 ppm (3H), 1,5 ppm (2H), 3,2 ppm (2H), 4,0 ppm (0,8 H, br), 4,2 ppm (1H), 6,8 ppm (1H).

### Beispiele 2 bis 4

### Herstellung weiterer N-n-Alkylmilchsäureamide

Entsprechend Beispiel 1 werden Milchsäure-N-hexylamid, Milchsäure-N-heptylamid und Milchsäure-N-nonylamid durch Amidierung von Ethyllactat mit n-Hexylamin, n-Heptylamin bzw. n-Nonylamin hergestellt.

### Beispiel 5

### Herstellung von N-(2-Ethylhexyl)-milchsäureoctylamid

Entsprechend Beispiel 1 wird Milchsäure-N-(2-ethylhexylamid) durch Amidierung von Ethyllactat mit 2-Ethylhexylamin hergestellt.

### Beispiel 6

### Herstellung von Glycolsäure-N-octylamid

0,75 mol Glycolsäure werden bei 80°C geschmolzen, nachfolgend werden 1,11 mol Octylamin unter Rühren zugetropft. Nach Abklingen der exothermen Reaktion wird für eine Stunde auf 165°C erhitzt und nachfolgend das überschüssige Amin im Vakuum destilliert. Das Produkt wird nach Abkühlen in Form eines weißen, kristallinen Feststoffes erhalten.

### Beispiel 7

### Herstellung von 4-Hydroxybuttersäure-N-octylamid

1 mol Octylamin wird unter Rühren auf 100°C erhitzt. 1 mol gamma-Butyrolacton wird über einen Zeitraum von 30 Minuten zugetropft. Das Gemisch wird für drei Stunden auf 120°C erhitzt. Zum Ende der Reaktion wird ein Vakuum von 50 mbar angelegt. Das Produkt wird nach Abkühlen in Form eines schwach gelben Feststoffes erhalten.

### Beispiel 8

### Herstellung von PEG-3-Milchsäureoctylamid

100 g Milchsäure-N-octylamid werden in einem Druckreaktor mit 1 g 45 % KOH versetzt und eine Stunde bei 115°C und vermindertem Druck getrocknet. Anschließend werden bei 130°C über 40 Minuten 3 mol-Äquivalente Ethylenoxid zudosiert und das Produkt für eine Nachreaktionszeit von 45 Minuten weiter bei dieser Temperatur gehalten. Nach Abkühlen auf Raumtemperatur wird mit Milchsäure neutralisiert. Das Produkt wird in Form einer klaren, bräunlichen Flüssigkeit erhalten. Mittels ¹H-NMR Analyse wird eine Anlagerung von 3,1 mol Ethylenoxid pro mol Milchsäureoctylamid bestimmt.

### Beispiel 9

### Herstellung von PEG-10-Milchsäureoctylamid

100 g Milchsäure-N-octylamid werden in einem Druckreaktor mit 1 g 45 % KOH versetzt und eine Stunde bei 115°C und vermindertem Druck getrocknet. Anschließend werden bei 130°C über 70 Minuten 10 mol-Äquivalente Ethylenoxid zudosiert und das Produkt für eine Nachreaktionszeit von 60 Minuten weiter bei dieser Temperatur gehalten. Nach Abkühlen auf Raumtemperatur wird mit Milchsäure neutralisiert. Mittels ¹H-NMR Analyse wird eine Anlagerung von 10,9 mol Ethylenoxid pro mol Milchsäureoctylamid bestimmt.

### Beispiel 10

### Antimikrobielle Wirkung

20 ml einer 0,3 Gew-% Lösung der unten in der Tabelle aufgeführten Testsubstanz in Wasser wird mit 200 µl einer frischen 48 Stunden-Kultur von C. xerosis oder S. epidermidis inokuliert und drei Tage bei 37°C bebrütet. Direkt nach der Zugabe sowie nach 1, 3, 24 und 48 Stunden werden Proben von je 1 ml entnommen und die Keimzahl bestimmt.

| | | KBE / ml Stunden | | | | |
|---|---|---|---|---|---|---|
| Testsubstanz | Mikroorganismus | 0 | 1 | 3 | 24 | 48 |
| Milchsäureoctylamid | | | | | | |
| | C. xerosis | 9,1E+5 | 1,5E+4 | < 2,0E+2 | < 2,0E+2 | < 2,0E+2 |
| | S. epidermidis | 1, 9E+7 | 3,7E+6 | 3,0E+6 | 2,3E+6 | 8,5E+5 |
| Milchsäurehexylamid | | | | | | |
| | C. xerosis | 2,5E+6 | 5,5E+5 | 2,6E+3 | < 2,0E+2 | < 2,0E+2 |
| | S. epidermidis | 8,6E+6 | 1,7E+6 | 7,1E+5 | < 2,0E+2 | < 2,0E+2 |
| PEG-3-Milchsäureoctylamid | | | | | | |
| | C. xerosis | 9,1E+5 | 6,3E+4 | 3,7E+3 | < 2,0E+2 | < 2,0E+2 |
| | S. epidermidis | 1, 9E+7 | 4,1E+6 | 3,1E+6 | < 2,0E+2 | < 2,0E+2 |
| PEG-10-Milchsäureoctylamid | | | | | | |
| | C. xerosis | 9,1E+5 | 4,5E+5 | 1,8E+5 | 1,6E+3 | < 2,0E+2 |
| | S. epidermidis | 1, 9E+7 | 2,3E+6 | 9,3E+6 | 8,1E+4 | < 2,0E+2 |

### Beispiel 11

### pH-Abhängigkeit der antimikrobiellen Wirkung

Entsprechend Beispiel 8 wird die antimikrobielle Wirkung von Milchsäureoctylamid in gepufferten Lösungen bei verschiedenen pH-Werten überprüft.

| | | KBE / ml Stunden | | | | |
|---|---|---|---|---|---|---|
| Substanz | Mikroorganismus | 0 | 1 | 3 | 24 | 48 |
| Milchsäureoctylamid, pH 4,5 | | | | | | |
| | C. xerosis | 1,0E+6 | 5,5E+4 | < 2,0E+2 | < 2,0E+2 | < 2,0E+2 |
| | S. epidermidis | 3,7E+6 | 2,1E+6 | 7,9E+4 | < 2,0E+2 | < 2,0E+2 |
| Milchsäure-octylamid, pH 5,5 | | | | | | |
| | C. xerosis | 1,0E+6 | 1,8E+5 | 3,5E+4 | < 2,0E+2 | < 2,0E+2 |
| | S. epidermidis | 3,7E+6 | 1,7E+6 | 4,7E+3 | < 2,0E+2 | < 2,0E+2 |
| Milchsäureoctylamid, pH 6,9 | | | | | | |
| | C. xerosis | 7,3E+5 | 3,3E+3 | < 2,0E+2 | < 2,0E+2 | < 2,0E+2 |
| | S. epidermidis | 3,1E+6 | 1,5E+6 | 5,7E+4 | < 2,0E+2 | < 2,0E+2 |

### Beispiel 12

### Pumpspray-Deoformulierungen ohne Ethanol

| | PS-1 | PS-2 | PS-3 | PS-4 | PS-5 | PS-6 | PS-7 | PS-8 |
|---|---|---|---|---|---|---|---|---|
| Phase A | | | | | | | | |
| Beispiel 1 | 0,3 | 1,0 | | | 0,3 | 1,0 | | |
| Beispiel 8 | | | 0,3 | 1,0 | | | 0,3 | 1,0 |
| TEGO® SML 20 | 3,0 | 4,0 | 3,0 | 4,0 | 3,0 | 4,0 | 3,0 | 4,0 |
| Fragrance | 0,5 | 0,5 | 0,5 | 0,5 | 0,5 | 0,5 | 0,5 | 0,5 |
| Ethanol | | | | | | | | |
| ABIL® B 8832 | 0,5 | 0,5 | 0,5 | 0,5 | 0,5 | 0,5 | 0,5 | 0,5 |
| Tegosoft® M | | | | | | | | |

| Phase B | | | | | | | | |
|---|---|---|---|---|---|---|---|---|
| Water | ad 100 | ad 100 | ad 100 | ad 100 | ad 100 | ad 100 | ad 100 | ad 100 |
| Allantoin | 0,2 | 0,2 | 0,2 | 0,2 | 0,2 | 0,2 | 0,2 | 0,2 |
| Panthenol | 0,1 | 0,1 | 0,1 | 0,1 | 0,1 | 0,1 | 0,1 | 0,1 |
| Tegosoft® GC | 1,0 | 1,0 | 1,0 | 1,0 | 1,0 | 1,0 | 1,0 | 1,0 |

| Phase C | | | | | | | | |
|---|---|---|---|---|---|---|---|---|
| Reach® 501 | - | - | - | - | 30,0 | 30,0 | 30,0 | 30,0 |
| Kathon^{™} CG | 0,03 | 0,03 | 0,03 | 0,03 | 0,03 | 0,03 | 0,03 | 0,03 |

### Beispiel 13

Pumpspray-Deoformulierungen mit Ethanol

| | PS-9 | PS-10 | PS-11 | PS-12 | PS-13 | PS-14 | PS-15 | PS-16 |
|---|---|---|---|---|---|---|---|---|
| Phase A | | | | | | | | |
| Beispiel 1 | 0,3 | 1,0 | - | - | 0,3 | 1,0 | - | - |
| Beispiel 8 | - | - | 0,3 | 1,0 | - | - | 0,3 | 1,0 |
| TEGO® SML 20 | 3,0 | 4,0 | 3,0 | 4,0 | 3,0 | 4,0 | 3,0 | 4,0 |
| Fragrance | 0,5 | 0,5 | 0,5 | 0,5 | 0,5 | 0,5 | 0,5 | 0,5 |
| Ethanol | ad 100 | ad 100 | ad 100 | ad 100 | 30,0 | 30,0 | 30,0 | 30,0 |
| ABIL® B 8832 | - | - | - | - | - | - | - | - |
| Tegosoft® M | 1 | 1 | 1 | 1 | - | - | - | - |
| | | | | | | | | |

| Phase B | | | | | | | | |
|---|---|---|---|---|---|---|---|---|
| Water | - | - | - | - | ad 100 | ad 100 | ad 100 | ad 100 |
| Allantoin | - | - | - | - | - | - | - | - |
| Panthenol | - | - | - | - | - | - | - | - |
| Tegosoft® GC | - | - | - | - | - | - | - | - |
| | | | | | | | | |

| Phase C | | | | | | | | |
|---|---|---|---|---|---|---|---|---|
| Reach® 501 | - | - | - | - | 20,0 | 20,0 | 20,0 | 20,0 |
| Kathon^{™} CG | 0,03 | 0,03 | 0,03 | 0,03 | 0,03 | 0,03 | 0,03 | 0,03 |

### Beispiel 14

### Roll-on Deoformulierungen ohne Ethanol

| | RO-1 | RO-2 | RO-3 | RO-4 | RO-5 | RO-6 | RO-7 | RO-8 |
|---|---|---|---|---|---|---|---|---|
| Phase A | | | | | | | | |
| Beispiel 1 | 0,3 | 1,0 | - | - | 0,3 | 1,0 | - | - |
| Beispiel 8 | - | - | 0,3 | 1,0 | - | - | 0,3 | 1,0 |
| TEGO® SML 20 | 3,0 | 4,0 | 3,0 | 4,0 | 3,0 | 4,0 | 3,0 | 4,0 |
| Fragrance | 0,5 | 0,5 | 0,5 | 0,5 | 0,5 | 0,5 | 0,5 | 0,5 |
| Ethanol | - | - | - | - | - | - | - | - |
| ABIL® B 8832 | 1,0 | 1,0 | 1,0 | 1,0 | 1,0 | 1,0 | 1,0 | 1,0 |
| Tegosoft® GC | - | - | - | - | - | - | - | - |
| | | | | | | | | |

| Phase B | | | | | | | | |
|---|---|---|---|---|---|---|---|---|
| Water | ad 100 | ad 100 | ad 100 | ad 100 | ad 100 | ad 100 | ad 100 | ad 100 |
| Walocel® | - | - | - | - | - | - | - | - |
| HM 4000 | 0,7 | 0,7 | 0,7 | 0,7 | 0,7 | 0,7 | 0,7 | 0,7 |
| Tylose® H4000 | - | - | - | - | - | - | - | - |
| | | | | | | | | |
| Water (for swelling) | 34,3 | 34,3 | 34,3 | 34,3 | 34,3 | 34,3 | 34,3 | 34,3 |
| Allantoin | - | - | - | - | 0,2 | 0,2 | 0,2 | 0,2 |
| Panthenol | - | - | - | - | 0,1 | 0,1 | 0,1 | 0,1 |
| | | | | | | | | |

| Phase C | | | | | | | | |
|---|---|---|---|---|---|---|---|---|
| Reach® 501 | - | - | - | - | 20,0 | 20,0 | 20,0 | 20,0 |
| Kathon^{™} CG | 0,03 | 0,03 | 0,03 | 0,03 | 0,03 | 0,03 | 0,03 | 0,03 |

### Beispiel 15

### Roll-on Deoformulierungen mit Ethanol

| | RO-9 | RO-10 | RO-11 | RO-12 | RO-13 | RO-14 | RO-15 | RO-16 |
|---|---|---|---|---|---|---|---|---|
| Phase A | | | | | | | | |
| Beispiel 1 | 0,3 | 1,0 | - | - | 0,3 | 1,0 | - | - |
| Beispiel 8 | - | - | 0,3 | 1,0 | - | - | 0,3 | 1,0 |
| TEGO® SML 20 | 3,0 | 4,0 | 3,0 | 4,0 | 3,0 | 4,0 | 3,0 | 4,0 |
| Fragrance | 0,5 | 0,5 | 0,5 | 0,5 | 0,5 | 0,5 | 0,5 | 0,5 |
| Ethanol | 20,0 | 20,0 | 20,0 | 20,0 | 20,0 | 20,0 | 20,0 | 20,0 |
| ABIL® B 8832 | 0,5 | 0,5 | 0,5 | 0,5 | 0,5 | 0,5 | 0,5 | 0,5 |
| Tegosoft® GC | 1,0 | 1,0 | 1,0 | 1,0 | 1,0 | 1,0 | 1,0 | 1,0 |
| | | | | | | | | |

| Phase B | | | | | | | | |
|---|---|---|---|---|---|---|---|---|
| Water | ad 100 | ad 100 | ad 100 | ad 100 | ad 100 | ad 100 | ad 100 | ad 100 |
| Walocel® | - | - | - | - | - | - | - | - |
| HM 4000 | 0,7 | 0,7 | 0,7 | 0,7 | - | - | - | - |
| Tylose® H4000 | - | - | - | - | 0,75 | 0,75 | 0,75 | 0,75 |
| | | | | | | | | |
| Water (for swelling) | 36,75 | 36,75 | 36,75 | 36,75 | 36,75 | 36,75 | 36,75 | 36,75 |
| Allantoin | 0,1 | 0,1 | 0,1 | 0,1 | 0,1 | 0,1 | 0,1 | 0,1 |
| Panthenol | 0,2 | 0,2 | 0,2 | 0,2 | 0,2 | 0,2 | 0,2 | 0,2 |

| Phase C | | | | | | | | |
|---|---|---|---|---|---|---|---|---|
| Reach® 501 | - | - | - | - | - | - | - | - |
| Kathon^{™} CG | 0,03 | 0,03 | 0,03 | 0,03 | 0,03 | 0,03 | 0,03 | 0,03 |

| | RO-17 | RO-18 | RO-19 | RO-20 | RO-21 | RO-22 | RO-23 | RO-24 |
|---|---|---|---|---|---|---|---|---|
| Phase A | | | | | | | | |
| Beispiel 1 | 0,3 | 1,0 | | | 0,3 | 1,0 | | |
| Beispiel 8 | | | 0,3 | 1,0 | | | 0,3 | 1,0 |
| TEGO® SML 20 | 3,0 | 4,0 | 3,0 | 4,0 | 3,0 | 4,0 | 3,0 | 4,0 |
| Fragrance | 0,5 | 0,5 | 0,5 | 0,5 | 0,5 | 0,5 | 0,5 | 0,5 |
| Ethanol | 20,0 | 20,0 | 20,0 | 20,0 | 20,0 | 20,0 | 20,0 | 20,0 |
| ABIL® B 8832 | 0,5 | 0,5 | 0,5 | 0,5 | 0,5 | 0,5 | 0,5 | 0,5 |
| Tegosoft® GC | 1,0 | 1,0 | 1,0 | 1,0 | 1,0 | 1,0 | 1,0 | 1,0 |

| Phase B | | | | | | | | |
|---|---|---|---|---|---|---|---|---|
| Water | ad 100 | ad 100 | ad 100 | ad 100 | ad 100 | ad 100 | ad 100 | ad 100 |
| Walocel | 0,7 | 0,7 | 0,7 | 0,7 | - | - | - | - |
| | | | | | | | | |
| Hydroxyethyl cellulose | - | - | - | - | 0,75 | 0,75 | 0,75 | 0,75 |
| | | | | | | | | |
| Water (for swelling) | 36,75 | 36,75 | 36,75 | 36,75 | 36,75 | 36,75 | 36,75 | 36,75 |
| Allantoin | 0,1 | 0,1 | 0,1 | 0,1 | 0,1 | 0,1 | 0,1 | 0,1 |
| Panthenol | 0,2 | 0,2 | 0,2 | 0,2 | 0,2 | 0,2 | 0,2 | 0,2 |

| Phase C | | | | | | | | |
|---|---|---|---|---|---|---|---|---|
| Reach® 501 | 20,0 | 20,0 | 20,0 | 20,0 | 20,0 | 20,0 | 20,0 | 20,0 |
| Kathon^{™} CG | 0,03 | 0,03 | 0,03 | 0,03 | 0,03 | 0,03 | 0,03 | 0,03 |

### Beispiel 16

### Pumpspray-Emulsionen

| | PE-1 | PE-2 | PE-3 | PE-4 | PE-5 | PE-6 | PE-7 | PE-8 |
|---|---|---|---|---|---|---|---|---|
| Phase A | | | | | | | | |
| Beispiel 1 | 0,3 | 1,0 | - | - | 0,3 | 1,0 | - | - |
| Beispiel 8 | - | - | 0,3 | 1,0 | - | - | 0,3 | 1,0 |
| TEGO® SML 20 | 3,0 | 4,0 | 3,0 | 4,0 | 3,0 | 4,0 | 3,0 | 4,0 |
| Teginacid® H | 3,0 | 3,0 | 3,0 | 3,0 | 3,0 | 3,0 | 3,0 | 3,0 |
| TEGO® Alkanol 18 | 1,0 | 1,0 | 1,0 | 1,0 | 1,0 | 1,0 | 1,0 | 1,0 |
| ABIL® 350 | 0,5 | 0,5 | 0,5 | 0,5 | 0,5 | 0,5 | 0,5 | 0,5 |
| Tegosoft® Liquid | 4,0 | 4,0 | 4,0 | 4,0 | 4,0 | 4,0 | 4,0 | 4,0 |
| Tegosoft® CT | 4,0 | 4,0 | 4,0 | 4,0 | 4,0 | 4,0 | 4,0 | 4,0 |
| | | | | | | | | |

| Phase B | | | | | | | | |
|---|---|---|---|---|---|---|---|---|
| Water | ad 100 | ad 100 | ad 100 | ad 100 | ad 100 | ad 100 | ad 100 | ad 100 |
| Glycerol | 3,0 | 3,0 | 3,0 | 3,0 | 3,0 | 3,0 | 3,0 | 3,0 |
| | | | | | | | | |

| Phase C | | | | | | | | |
|---|---|---|---|---|---|---|---|---|
| Reach® 501 | - | - | - | - | 20,0 | 20,0 | 20,0 | 20,0 |
| Kathon^{™} CG | 0,03 | 0,03 | 0,03 | 0,03 | 0,03 | 0,03 | 0,03 | 0,03 |
| Fragrance | 0,5 | 0,5 | 0,5 | 0,5 | 0,5 | 0,5 | 0,5 | 0,5 |

### Beispiel 17

### Roll-on Emulsionen

| | RE-1 | RE-2 | RE-3 | RE-4 | RE-7 | RE-8 | RE-9 | RE-10 |
|---|---|---|---|---|---|---|---|---|
| Phase A | | | | | | | | |
| Beispiel 1 | 0,3 | 1,0 | - | - | 0,3 | 1,0 | - | - |
| Beispiel 8 | - | - | 0,3 | 1,0 | - | - | 0,3 | 1,0 |
| TEGO® SML 20 | - | - | - | - | - | - | - | - |
| TEGO® Alkanol S2P | 2,2 | 2,2 | 2,2 | 2,2 | 2,2 | 2,2 | 2,2 | 2,2 |
| TEGO® Alkanol S20P | 1,0 | 1,0 | 1,0 | 1,0 | 1,0 | 1,0 | 1,0 | 1,0 |
| Tegosoft® Liquid | 2,0 | 2,0 | 2,0 | 2,0 | 2,0 | 2,0 | 2,0 | 2,0 |
| Varonic® APS | 2,0 | 2,0 | 2,0 | 2,0 | 2,0 | 2,0 | 2,0 | 2,0 |
| ABIL® 350 | 0,5 | 0,5 | 0,5 | 0,5 | 0,5 | 0,5 | 0,5 | 0,5 |
| | | | | | | | | |

| Phase B | | | | | | | | |
|---|---|---|---|---|---|---|---|---|
| Water | ad 100 | ad 100 | ad 100 | ad 100 | ad 100 | ad 100 | ad 100 | ad 100 |
| Glycerol | 3,0 | 3,0 | 3,0 | 3,0 | 3,0 | 3,0 | 3,0 | 3,0 |
| | | | | | | | | |

| Phase C | | | | | | | | |
|---|---|---|---|---|---|---|---|---|
| Reach® 501 | - | - | - | - | 30,0 | 30,0 | 30,0 | 30,0 |
| Kathon^{™} CG | 0,03 | 0,03 | 0,03 | 0,03 | 0,03 | 0,03 | 0,03 | 0,03 |
| Fragrance | 0,5 | 0,5 | 0,5 | 0,5 | 0,5 | 0,5 | 0,5 | 0,5 |

### Beispiel 18

### Deosticks ohne ACH

| | ST-1 | ST-2 | ST-3 | ST-4 |
|---|---|---|---|---|
| Phase A | | | | |
| Varonic® APM | 77,5 | 77,5 | - | - |
| Propylene Glycol | 10,0 | 10,0 | 28,5 | 28,5 |
| Butylene Glycol | - | - | 20,0 | 20,0 |
| Sodium Stearate | 8,0 | 8,0 | 8,0 | 8,0 |
| Water | 3,0 | 3,0 | 8,0 | 8,0 |

| Phase B | | | | |
|---|---|---|---|---|
| Fragrance | 1,0 | 1,0 | 1,0 | 1,0 |
| Beispiel 1 | 0,5 | - | 0,5 | - |
| Beispiel 8 | - | 0,5 | - | 0,5 |
| Water | ad 100 | ad 100 | ad 100 | ad 100 |
| Ethanol | - | - | 34,0 | 34,0 |

### Beispiel 19

### Deosticks mit ACH

| | ST-5 | ST-6 |
|---|---|---|
| Phase A | | |
| TEGO® Alkanol 18 | 23,5 | 23,5 |
| Hydrogenated | | |
| Castor Oil | 4,0 | 4,0 |
| Tegosoft® PBE | 10,0 | 10,0 |
| Tegosoft® P | 16,0 | 16,0 |
| TEGO® Alkanol L4 | 1,0 | 1,0 |

| Phase B | | |
|---|---|---|
| Cyclopentasiloxane | 20,0 | 20,0 |

| Phase C | | |
|---|---|---|
| Micro-Dry® Ultrafine | 20,0 | 20,0 |

| Phase D | | |
|---|---|---|
| Fragrance | 1,0 | 1,0 |
| Beispiel 1 | 0,5 | |
| Beispiel 8 | | 0,5 |

## Patentansprüche

1. Verbindungen der allgemeinen Formel (I) 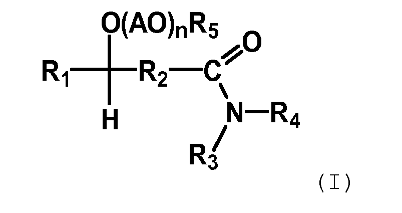 worin die Substituenten und Indices die folgende Bedeutung haben:
R₁ = H oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen,
R₂ = direkte Bindung oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen,
R₃, R₄= unabhängig voneinander H oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen,
R₅ = H oder C₁-C₄-Acylrest,
AO = mindestens ein Rest, ausgesucht aus der Gruppe -CH₂CH₂O-, -CH₂CHR₆O-, -CHR₆CH₂O-, -CH₂CH₂OHCH₂O- mit R₆=C₁-C₄-Alkyl,
n = 1 bis 20, bezogen auf den Mittelwert aller Oligomere,
mit der Maßgabe, dass R₃ und R₄ nicht gleichzeitig H sind und die Summe der C-Atome in R₃ und R₄ nicht größer als 18 ist und dass die Summe der C-Atome in R₁, R₂, R₃ und R₄ 36 nicht überschreitet.

2. Verbindungen gemäß Anspruch 1, **dadurch gekennzeichnet, dass** n = 1 bis 20, R₅=H, R₁=H oder CH₃, R₂ eine direkte Bindung oder ein gegebenenfalls Hydroxyalkylgruppen enthaltender Kohlenwasserstoffrest mit 1 bis 4 C-Atomen, R₃=H und R₄=C₄-C₁₂-Alkylrest ist.

3. Verbindungen gemäß mindestens einem der Ansprüche 1 oder 2, **dadurch gekennzeichnet, dass** AO=-CH₂CH₂O- und n = 2 bis 5 ist.

4. Verwendung von Verbindungen der allgemeinen Formel (Ia) 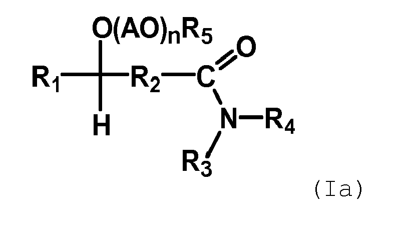
R₁ = H oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen,
R₂ = direkte Bindung oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen,
R₃, R₄ = unabhängig voneinander H oder ein gegebenenfalls verzweigter und/oder Mehrfachbindungen und/oder Hydroxygruppen und/oder Alkylgruppen und/oder Hydroxyalkylgruppen enthaltender Kohlenwasserstoffrest mit 1 bis 18 C-Atomen,
R₅ = H oder C₁-C₄-Acylrest,
AO = mindestens ein Rest, ausgesucht aus der Gruppe -CH₂CH₂O-, -CH₂CHR₆O-, -CHR₆CH₂O-, -CH₂CH₂OHCH₂Omit R₆=C₁-C₄Alkyl,
n = 0 bis 20, bezogen auf den Mittelwert aller Oligomere,
mit der Maßgabe, dass R₃ und R₄ nicht gleichzeitig H sind und die Summe der C-Atome in R₃ und R₄ nicht größer als 18 ist und dass die Summe der C-Atome in R₁, R₂, R₃ und R₄ 36 nicht überschreitet,
zur Kontrolle von Mikroorganismen.

5. Verwendung von Verbindungen gemäß Anspruch 4 zur selektiven Kontrolle von Mikroorganismen, **dadurch gekennzeichnet, dass** n = 0 bis 20, R₅=H, R₁=H oder CH₃, R₂ eine direkte Bindung oder ein gegebenenfalls Hydroxyalkylgruppen enthaltender Kohlenwasserstoffrest mit 1 bis 4 C-Atomen, R₃=H und R₄=C₄-C₁₂-Alkylrest ist.

6. Verbindungen gemäß mindestens einem der Ansprüche 4 oder 5, **dadurch gekennzeichnet, dass** AO=-CH₂CH₂O- und n = 2 bis 5 ist.

7. Verwendung gemäß mindestens einem der Ansprüche 4 bis 6 zur selektiven Kontrolle von Mikroorganismen, **dadurch gekennzeichnet, dass** es sich bei den Mikroorganismen um grampositive, insbesondere coryneforme, Bakterien handelt.

8. Formulierungen zur selektiven Kontrolle von Mikroorganismen, enthaltend mindestens eine Verbindung der allgemeinen Formel (Ia).

9. Formulierungen gemäß Anspruch 8, **dadurch gekennzeichnet, dass** sie 0,001 bis 10 Gew.-% mindestens einer Verbindung der allgemeinen Formel (Ia) enthalten.

10. Formulierungen gemäß einem der Ansprüche 8 und 9, **dadurch gekennzeichnet, dass** es sich um Desodorantien in Form von Aerosolsprays, Pumpsprays, Roll-On Formulierungen, Deo-Sticks, W/O- oder O/W-Emulsionen oder Wischtüchern handelt.

11. Verwendung von Formulierungen gemäß einem der Ansprüche 8 und 9 zur Bekämpfung von Akne.

12. Verwendung von Formulierungen gemäß einem der Ansprüche 8 und 9 zur Bekämpfung von Schuppen.

13. Formulierungen gemäß einem der Ansprüche 8 und 9, **dadurch gekennzeichnet, dass** es sich um Mundhygieneprodukte und insbesondere um Mundspüllösungen oder Zahnpasten handelt.
